# EUROPEAN PATENT SPECIFICATION

(11) **EP 0 659 402 B1**
(45) Date of publication and mention of the grant of the patent: **13.03.2002**
(21) Application number: 94107676.2
(22) Date of filing: 18.05.1994
(51) Int. Cl.: A61K 7/48, A61K 31/35, A61K 31/12, A61K 47/48, A23L 3/3499, A23L 3/3544

(54) **Formulations containing carotenoids and procarotenoids combined with polyphenols in the prevention of the damages due to an abnormal production of free radicals**
Karotenoide und Prokarotenoide enthaltende Zusammensetzungen kombiniert mit Polyphenolen zur Vorbeugung von durch abnormale freie Radikalbildung verursachten Schäden
Compositions contenant de caroténoides et de procaroténoides en association avec des polyphénoles pour la prévention de dommages provoqués par la production anormale des radicaux libres

(30) Priority: 21.12.1993 IT MI932688
(43) Date of publication of application: 28.06.1995
(73) Proprietor: INDENA S.p.A., 20141 Milano (IT)
(72) Inventor: Bombardelli, Ezio, I-20141 Milano (IT); Morazzoni, Paolo, I-20141 Milano (IT)
(74) Representative: Minoja, Fabrizio, Dr.

(56) References cited:
- EP-A- 0 511 895
- WO-A-92/12702
- WO-A-94/09801
- FR-M- 5 097
- GB-A- 2 174 904
- GB-A- 2 178 314
- DATABASE WPI Week 9410 Derwent Publications Ltd., London, GB; AN 94-080695 XP002010127 & RU-C-2 002 438 (PILIPCHENKO L.N.) , 15 November 1993
- DATABASE WPI Week 9327 Derwent Publications Ltd., London, GB; AN 93-216669 XP002010128 & JP-A-05 139 987 (SKY FOOD KK) , 8 June 1993
- DATABASE WPI Week 9403 Derwent Publications Ltd., London, GB; AN 94-021900 XP002010129 & JP-A-05 328 947 (SANKAINO CHINMI KK) , 14 December 1993

## Description

The present invention relates to novel formulations and combinations of lipophilic and hydrophilic antioxidants and the use thereof in the therapeutic, foodstuff and cosmetic fields. These formulations are based on the use of lycopene, with polyphenols of catechic and flavanolignane structures, both pure and contained in extracts.

It is well established in literature that the administration of vitamin E, β-carotene, lycopene and ubidecarenone (Coenzyme Q 10) through the diet or in pharmaceutical and nutritional formulations significantly decreases the incidence of cardiovascular diseases, and also it seems to play an important role in the prevention of some tumours. Dietetic and pharmaceutical compositions comprising both lipophilic and hydrophilic antioxidant compounds are known from EP 0 511 895 and JP-A-05139987. As far as the anti-aterosclerotic activity of carotenoids is concerned, according to recent studies, the capability of carotenoids to prevent lipoproteins oxidation (therefore interfering with the vasal uptake thereof) appears to be one of the most important mechanisms. In fact, carotenoids are incorporated physiologically in low density lipoproteins (LDL) and, preventing the oxidation thereof, they effectively counteract the primum movens of the atherosclerotic damage, which occurs mainly to the detriment of endothelial tissue [1-4].

Carotenoids are effective antioxidants at the cell level, their function taking place particularly on the cell in active proliferation, in which the frequency of genic error is higher. In support of carotenoids bioavailability, the preventive action thereof, after oral administration, against skin damages due to ultraviolet radiations, which are known to give rise to oxygen radicals, as well as the role thereof at the peripheral level on the eye functions, are well known.

Polyphenol substances having a catechic structure of the dimeric and oligomeric types are widely used in cardiovascular therapy and in ophthalmology due to their action on large and small calibre vessels. These natural polyphenols exert in fact a beneficial modulating action on capillary fragility and permeability, as well as on the protection of endotheliums. Recent literature agrees in considering the antioxidant activity an important mechanism at the base of the biological effect of said compounds.

Flavanolignanes, among which are silymarin and its three main components (silybin, silydianin and silychristin), are characterized by a marked antiradical power at the hepatic and peripheral levels. As recently proved by the Applicant for the procyanidole oligomers extracted from Vitis Vinifera [5] and by other authors for a number of polyphenolic substances extracted from different vegetable sources [6, 7], another interesting biological property of this series of compounds is that they have a remarkable antimutagenic action. Such a characteristic, which is partly connected with the antioxidant effect, takes place both on spontaneous mutagenesis and on the one induced either by ultraviolet radiations or by mutagenic products such as medicaments or atmospheric pollutants.

The action sites of the polyphenolic substances are very different from those of carotenoids and analogues thereof, therefore the concomitant administration of lycopene and hydrophilic compounds gives rise to an unexpected, surprising advantage from the biological point of view.

Carotenoids such as vitamin E, β-carotene, lycopene, coenzyme Q10 and isomers thereof proved to be very effective antiradicalic compounds against the activated forms of oxygen, both in the conventional tests of lipid peroxidation, such as the xanthine-xanthine oxidase system, and in a very selective model, recently prepared by the Applicant, which involves lipid peroxidation of an unsaturated phospholipid in an aqueous medium by means of ultrasounds [8]. In the same tests, the polyphenols of the invention turned out to have the same or a higher activity than the prior art compounds.

The results obtained from these tests, using some polyphenolic substances and the lipophilic antioxidants Vitamin E and lycopene, are summarized in Tables 1 and 2.

**Table 1 -**

| Scavenger activity against the OH° hydroxy radical - Inductive phase. | |
|---|---|
| Substances | CI₅₀ |
| Proanthocyanidin A2 | 1.7 X 10⁻⁷ |
| 90% Procyanidole oligomers from Vitis vinifera | 1.1 X 10⁻⁷ |
| Silymarin | 1.2 X 10⁻⁷ |
| Vitamin E | 1.5 X 10⁻⁶ |
| Lycopene | 2.1 X 10⁻⁶ |

**Table 2 -**

| Scavenger activity against R°, ROO° lipid radicals. Propagation phase. | |
|---|---|
| Substances | CI₅₀ |
| Proanthocyanidin A2 | 4.0 X 10⁻⁷ |
| 90% Procyanidole oligomers from Vitis vinifera | 5.1 X 10⁻⁷ |
| Silymarin | 5.2 X 10⁻⁷ |
| Vitamin E | 1.0 X 10⁻⁷ |
| Lycopene | 1.8 X 10⁻⁷ |
| Lycopene + Proc. olig. from Vitis vinifera 1:4 | 1.2 X 10⁻⁹ |

It has surprisingly been found, and it is one object of the present invention, that the combination of an hydrophilic antioxidant selected from silymarin or one of the components thereof (silybin, silydianin, silychristin and isosilybin); proanthocyanidin A2; procyanidole oligomers extracted from Vitis vinifera or Camelia sinensis; extracts of Aesculus hippocastanum, Ginkgo biloba or Cardus marianum. with lycopene exerts an antioxidant action far greater than that of the single compounds tested at equal concentrations (Table 2).

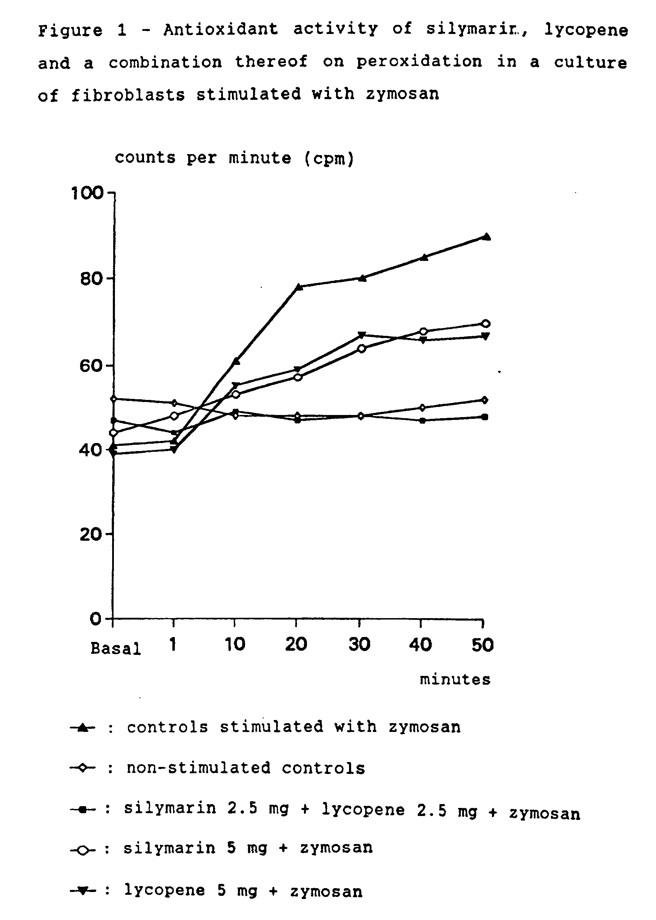

This finding was confirmed in another in vitro model which uses human fibroblasts stimulated with zymosan and evaluation of the peroxidative process by chemoluminescence. The combination of lycopene and silymarin, for example, proved to have an antioxidant effect surprisingly more marked than the single components (Fig. 1).

The results obtained in the in vitro models using pure lycopene were subsequently confirmed by in vivo models in which the use of combinations of lipophilic antioxidants and hydrophilic antioxidants gave an effective, unpredictable improvement on the experimental damages in which free radicals play an important role.

As a control, we selected in fact an inflammatory process model in which an irritating agent (phorbol ester) is used, which triggers off lipid peroxidation at the level of biological membranes and subsequently propagates through a radical chain reaction.

Table 3 shows the data of the antioedemigenic activity in the mouse, obtained with silymarin, silymarin combined with a lipophilic fraction having a high lycopene content (5%), the fraction being prepared by hexane extraction of dried skins of Licopersicum aesculentum and the fraction with lycopene. Data are expressed as percent decrease of the oedema measured at the 6th hour after the induction.

**Table 3 -**

| Anti-oedemigenic activity of silymarin, lycopene and combinations thereof on the phorbol ester oedema in the mouse. | | | |
|---|---|---|---|
| Substances | Dose µg/mouse | Oedema | % decrease |
| Controls | -- | 8.1 ± 0.2 | -- |
| Silymarin | 300 | 6.3 ± 0.2 | 22.2 NS |
| | 150 | 7.3 ± 0.4 | 9.9 NS |
| 5% Lycopene | 200 | 6.8 ± 0.3 | 16.0 NS |
| | 100 | 7.5 ± 0.4 | 12.4 NS |
| 5% Lycopene +Silymarin | 200+100 | 1.2 ± 0.2* | 86.4 |
| 5% Lycopene +Silymarin | 100+50 | 5.1 ± 0.0* | 37.0 |
| average ± SD | | | |

| | | | |
|---|---|---|---|
| *p<0.01 | | | |

All the above reported data shcw that the combination of the two antioxidants of different polarity increases by far the effectiveness of the single compounds. Most likely, such synergism is due to the concomitant action on more radical species which gives rise to a nearly complete blockage of the peroxidative process.

The same phenomenon takes place when the antimutagenic effect of these combinations is checked, by which effect the antioxidant component plays a paramount role and which is of paramount importance due to the implications in the prevention of cancer and cardiovascular pathologies.

By way of example, in Table 4 the effect of polyphenols extracted form green tea, of lycopene and of a combination thereof is reported.

**Table 4 -**

| Anti-mutagenic activity of procyanidole oligomers extracted from green tea, lycopene and a combination thereof on the frequency of spontaneous mutation in Salmonella typhimurium. | | | | |
|---|---|---|---|---|
| Substance mg/plate | TA98 | | TA100 | |
| | -S9 | +S9 | -S9 | +S9 |
| 0 | 1.0 (41) | 1.0 (48) | 1.0 (163) | 1.0 (133) |
| Proc. olig. | | | | |
| 1.0 | 1.0 | 0.8 | 0.9 | 1.0 |
| 2.5 | 1.0 | 0.7 | 1.1 | 1.0 |
| 5.0 | 0.5 | 0.5 | 0.7 | 0.9 |
| | | | | |
| Lycopene | | | | |
| 0.2 | 1.0 | 0.9 | 0.9 | 0.8 |
| 0.4 | 0.8 | 0.8 | 1.0 | 0.9 |
| 0.5 | 0.7 | 0.7 | 0.9 | 0.8 |
| | | | | |
| Proc. olig. + Lycopene | | | | |
| 0.50+0.10 | 0.6 | 0.5 | 0.7 | 0.7 |
| 1.25+0.20 | 0.5 | 0.5 | 0.7 | 0.8 |
| 2.50+0.25 | 0.4 | 0.3 | 0.7 | 0.7 |

As the polyphenolic fraction from green tea, a product is used having an 85% polyphenol titre, expressed as epigallocatechin gallate, and a low caffeine content (<0.3%). Using mixtures of the lycopene fraction with tea polyphenols, inhibition of about 50% of spontaneous mutation was obtained in the Ames test (Salmonella typhimurium TA-100 and TA-98).

Such an effect turned out to be independent of the metabolic activation (-S9 or +S9). Analogous effects are obtained on Saccharomyces cerevisiae strains, where the effect on spontaneous mutation was controlled at the nuclear level and, even more important, at the level of mitochondrial information.

The ratios of the two fractions can vary from 1 part of polyphenol to 1 part of lycopene, depending on the field of application of the different formulations.

As excipients for use in these formulations, particularly important are phospholipids, both the natural and the synthetic ones, and the raw lecithins having different phospholipid contents. Phospholipids are a particularly suitable carrier to promote adsorption of polyphenolic substances both of catechic and flavanolignane nature. The preferred administration forms of these combinations are soft gelatin capsules, but hard gelatin capsules, cellulose or other matrixes as well as suppositories and transdermal forms can also be envisaged. The following examples further illustrate the invention.

### Example I - Formulation containing silymarin and lycopene in peanut oil

200 mg of lipophilic extract of Licopersicum aesculentum containing 5% of lycopene are mixed with 50 mg of silymarin, 50 mg of natural soy phosphatidylcholine and 50 mg of peanut oil; the products are encapsulated in soft gelatin capsules. The dose can vary from 1 to 5 capsules daily.

### Example II - Formulation containing procyanidole oligomers and lycopene in peanut oil

200 mg of lipophilic extract of Licopersicum aesculentum containing 5% of lycopene are mixed with 80 mg of procyanidole oligomers from Vitis vinifera, 50 mg of natural soy phosphatidylcholine and 50 mg of peanut oil; the products are encapsulated in soft gelatin capsules. The dose can vary from 1 to 5 capsules daily.

### Bibliography

1 - Ziegler R.G.
   A review of epidemiologic evidence that carotenoids reduce the risk of cancer
   J. Nutr. 119, 116-122, 1989
2 - Block G., Patterson B., Subar A.
   Fruits, vegetables and cancer prevention: a review of the epidemiological evidence
   Nutr. Cancer. 18, 1-29, 1992
3 - Frankel E.N., Kanner J., German J.B., Parks E., Kinsella J.E.
   Inhibition of oxidation of human low-density lipoprotein by phenolic substances in red wine
   The Lancet 341, 454-457, 1993
4 - Esterbauer H., Dieber-Rotheneder M., Waeg G., Striegl G., Jurgens G.
   Biochemical, structural and functional properties of oxidized low-density lipoproteins
   Chem. Rex. Toxicol. 3, 77-92, 1990
5 - Liviero L., Puglisi P.P., Bombardelli E., Morazzoni P., Aldini G.C., Carini M., Maffei-Facino R.
   Antimutagenic activity of antioxidant procyanidins from Vitis vinifera
   In: International Symposium of the Phytochemical Society of Europe - Phytochemistry of Plants used in Traditional Medicine, University of Lausanne, 29 September-1 October 1993. In press on Mutation Research.
6 - Hayatsu H., Arimoto S., Negishi T.
   Dietary inhibitors of mutagenesis and carcinogenesis.
   Mutation Res. 202, 429, 1988.
7 - Cassady J.M.
   Natural products as a source of potential cancer chemotherapeutic and chemopreventive agents.
   J. Nat. Prod. 53, 23, 1990.
8 - Maffei-Facino R., Carini M., Aldini G., Bombardelli E., Morazzoni P., Morelli R.
   Free radicals scavenging action and anti-enzyme activities of procyanidines from Vitis vinifera: a mechanism for their capillary protective action Arzneim. Forsch./Drug. Res., in press.

## Claims

1. Pharmaceutical, foodstuff, dietetic or cosmetic compositions containing as the active principle a lipophilic antioxidant in combination with an hydrophilic antioxidant, wherein the lipohilic antioxidant is lycopene, and the hydrophilic antioxidant is selected from: silymarin or one of the components thereof (silybin, silydianin, silychristin and isosilybin); proanthocyanidin A2; procyanidole oligomers extracted from Vitis vinifera or Camelia sinensis; extracts of Aesculus hippocastanum, Ginkgo biloba or Cardus marianum.

2. Compositions according to claim 1 containing as excipients natural or synthetic phospholipids, including raw lecithins having different phospholipid contents.

3. Compositions according to any one of the above claims, in form of soft or hard gelatin capsules, tablets, suppositories or transdermal forms.

## Patentansprüche

1. Pharmazeutische, Nahrungsmittel-, diätetische und kosmetische Zusammensetzungen, enthaltend als Wirkstoff ein lipophiles Antioxidans in Kombination mit einem hydrophilen Antioxidans, worin das lipophile Antioxidans Lycopin ist und das hydrophile Antioxidans ausgewählt ist unter: Silymarin oder einer von dessen Komponenten (Silybin, Silydianin, Silychristin und Isosilybin); Proanthocyanidin A2; Procyanidol-Oligomeren, extrahiert aus Vitis vinifera oder Camelia sinensis; Extrakten von Aesculus hippocastanum, Ginkgo biloba oder Cardus marianum.

2. Zusammensetzungen gemäß Anspruch 1, enthaltend als Exzipientien natürliche oder synthetische Phospholipide, einschließlich Rohlecithine mit verschiedenen Phospholipidgehalten.

3. Zusammensetzungen gemäß einem der vorhergehenden Ansprüche in Form von Weich- oder Hartgelatinekapseln, Tabletten, Suppositorien oder transdermalen Formen.

## Revendications

1. Compositions pharmaceutiques, alimentaires, diététiques ou cosmétiques contenant en tant que principe actif un antioxydant lipophile combiné avec un antioxydant hydrophile, dans lesquelles l'antioxydant lipophile est le lycopène, et l'antioxydant hydrophile est choisi parmi : la silymarine ou l'un des constituants de celle-ci (silybine, silydianine, silychristine et isosilybine) ; la proanthocyanidine A2 ; les oligomères de procyanidole extraits de *Vitis vinifera ou Camelia sinensis ; les extraits de Aesculus hippocastanum, Ginkgo biloba ou Cardus marianum.*

2. Compositions selon la revendication 1, contenant en tant qu'excipients des phospholipides naturels ou synthétiques, y compris des lécithines brutes ayant différentes teneurs en phospholipides.

3. Compositions selon l'une quelconque des revendications précédentes, sous la forme de capsules de gélatine molles ou dures, de comprimés, de suppositoires ou de formes transdermiques.
